Europäisches Patentamt

(19) European Patent Office    (11) Veröffentlichungsnummer: **0 045 915**
                                                         **B1**
Office européen des brevets

(12)                    **EUROPÄISCHE PATENTSCHRIFT**

(45) Veröffentlichungstag der Patentschrift:    �milli (51) Int. Cl.⁴: **A 61 B 5/10**
20.11.86

(21) Anmeldenummer: 81106034.2

(22) Anmeldetag: 31.07.81

(54) Fingerabdrucksensor zum Erzeugen eines dem topografischen Relief eines zu untersuchenden Fingers entsprechenden Ausgangssignals.

(30) Priorität: 11.08.80 US 176699

(43) Veröffentlichungstag der Anmeldung:
17.02.82 Patentblatt 82/7

(45) Bekanntmachung des Hinweises auf die Patenterteilung:
20.11.86 Patentblatt 86/47

(84) Benannte Vertragsstaaten:
AT BE CH DE FR GB IT LI NL SE

(56) Entgegenhaltungen:
FR - A - 1 355 839
FR - A - 2 407 530
US - A - 3 138 059
US - A - 3 882 463
US - A - 3 928 842
US - A - 3 982 836
US - A - 4 120 585

(73) Patentinhaber: **Siemens Aktiengesellschaft Berlin und München, Wittelsbacherplatz 2, D-8000 München 2 (DE)**

(72) Erfinder: **Rüll, Hartwig, Dr., Balduin-Helm-Strasse 39, D-8080 Fürstenfeldbruck (DE)**
Erfinder: **Devinney, Edward J., 100 Union Avenue, Delanco, New Jersey 08075 (US)**
Erfinder: **Chiu, Ming-Yee, Dr., 78 Boothby Drive, Mt. Laurel, New Jersey 08054 (US)**

**Beschreibung**

Die vorliegende Erfindung bezieht sich auf einen Fingerabdrucksensor gemäss dem Oberbegriff des Anspruchs 1 zum Erzeugen eines dem topografischen Relief eines zu untersuchenden Fingers entsprechenden Ausgangssignals.

Systeme zur Identifizierung eines Fingerabdrucks die den Abdruck eines auf eine Kontaktfläche gedrückten Fingers identifizieren, sind bekannt.

Beispielsweise geht aus der US-PS 4 053 228 ein Apparat zur Identifizierung eines Fingers hervor, der eine transparente Glasplatte aufweist, die als eine Kontaktfläche eines Fingerabdrucksensorlesers dient. Ein Fingerabdruck wird dadurch ausgebildet, dass der zu untersuchende Finger gegen die Rückfläche der Glasplatte gedrückt und in einer vorbestimmten Position gehalten wird. Der Fingerabdruck wird von einem die Vorderfläche der Glasplatte durchstrahlenden Lichtstrahl abgefragt. Der abfragende Strahl wird an der Rückseite teilweise reflektiert und erzeugt einen Signalstrahl, der die Fingerabdruckinformation trägt. Der reflektierte Signalstrahl wird dann mit einem Hologramm des gleichen Fingerabdrucks korreliert und dadurch die Identifizierung der Person ausgeführt.

Aus der US-PS 4 120 585 geht ein anderes System zur Identifizierung eines Fingerabdrucks hervor. Dieses System weist ein nachgiebiges optisches Prisma als Fingerabdrucksensor auf. Die Basis des Prismas wird durch den zu untersuchenden Finger der Person physisch berührt. Das elastische Prisma verformt sich unter dem ausgeübten Druck. Es reflektiert einen abtastenden Lichtstrahl teilweise auf eine fotoempfindliche Einrichtung, die dadurch aktiviert wird. Die fotoempfindliche Einrichtung aktiviert ihrerseits weitere optische Komponenten des Identifizierungssystems. Der Fingerabdrucksensor wird durch optische Einrichtungen hinsichtlich des Musters aus Vertiefungen und Erhöhungen des Fingerabdrucks einer zu identifizierenden Person überprüft.

Die bekannten Systeme zur Identifizierung eines Fingerabdrucks erfordern eine ziemlich aufwendige Technologie. Ein derartiges System sollte aber leicht zusammensetzbar und nicht teuer sein und gleichzeitig sollte es eine hohe Bildqualität der Fingerabdrücke liefern. Insbesondere sollte der Fingerabdrucksensor eine hohe Auflösung aufweisen.

Es ist die Aufgabe der Erfindung, einen Fingerabdrucksensor zu schaffen, der leicht zusammengebaut werden kann, nur geringe Kosten erfordert und der eine sehr grosse Empfindlichkeit und Auflösung aufweist.

Diese Aufgabe wird durch die im kennzeichnenden Teil des Patentanspruchs 1 angegebenen Merkmale gelöst.

Durch diese Lösung ist ein Fingerabdrucksensor geschaffen worden, der die Fingerabdruckinformation eines berührenden Fingers in ein lesbares optisches Bild und in ein elektrisches Ausgangssignal umwandelt.

Das elektrische Ausgangssignal des Fingerabdrucksensors repräsentiert die in dem Fingerabdruck enthaltene Information und kann zur Weiterverarbeitung in einen Rechner eingelesen werden.

Ein erfindungsgemässer Fingerabdrucksensor arbeitet sehr zuverlässig. Er erzeugt Bilder des Fingerabdrucks hoher Qualität. Die Bilder des Fingerabdrucks können digital verarbeitet werden. Laserabtasttechniken sind nicht erforderlich.

Bevorzugte und vorteilhafte Ausführungsformen der Erfindung gehen aus den Unteransprüchen 2 bis 5 hervor.

In Zusammenfassung weist ein Fingerabdrucksensor zur Erzeugung eines dem topografischen Relief eines zu untersuchenden Fingers entsprechenden Ausgangssignals einen aus einem transparenten elastischen Material gefertigten Kontaktkörper auf. Dieser Kontaktkörper weist eine Kontaktfläche zur Aufnahme des von einem zu untersuchenden Finger ausgeübten Kontaktdruckes auf. Wenn der Finger gegen die Kontaktfläche gedrückt wird, wird die Oberflächenstruktur entsprechend dem Muster des Fingerabdrucks verändert, und zwar aufgrund der elastischen Eigenschaften des gewählten Materials.

Der Fingerabdrucksensor weist ausserdem ein optisches Prisma, eine Lichtquelle und ein erstes optisches System auf. Das erste optische System führt einen ersten Lichtstrahl aus der Lichtquelle über das Prisma und das transparente elastische Material der Kontaktfläche des Kontaktkörpers zu. In Anwesenheit eines angedrückten Fingers reflektiert oder sendet die Kontaktfläche einen Lichtstrahl zurück, der entsprechend dem Muster des Fingerabdrucks moduliert ist.

Der Fingerabdrucksensor weist des weiteren ein zweites optisches System und einen Fotodetektor auf. Der Fotodetektor weist ein lichtempfindliches Feld zum Messen der Verteilung des auf es auftreffenden Lichtes auf. Das zweite optische System führt das von der Kontaktfläche reflektierte und das Prisma durchstrahlende modulierte Licht dem lichtempfindlichen Feld des Fotodetektors zu. Der Fingerabdrucksensor weist zudem einen mit dem Fotodetektor verbundenen Ausgang auf, dem das elektrische Ausgangssignal entnehmbar ist.

Das elastische Material, aus welchem die Kontaktfläche des Kontaktkörpers gebildet ist, kann ein Polymer sein. Als besonders geeignet haben sich Silikonkautschuk oder -gummi, ein Elastomer, erwiesen.

Der Kontaktkörper und das optische System sind aus zwei verschiedenen Elementen zusammengesetzt. Bei einer derartigen Ausführungsform ist eine aus einem transparenten und elastischen Material gebildete Sensorplatte vorgesehen. Diese Sensorplatte kann eine erste und zweite planare Seite aufweisen, welche Seiten parallel zueinander angeordnet sein können. Eine dieser Seiten kann als Kontaktfläche benutzt werden, auf die der Kontaktdruck ausgeübt wird, während die andere Seite einer Seite des Prismas zugewandt ist und parallel dazu verläuft.

Bevorzugte Ausführungsformen der Erfindung sind in den Figuren dargestellt und werden in der folgenden Beschreibung näher erläutert. Von den Figuren zeigen:

Fig. 1 eine Ausführungsform eines Fingerabdrucksensors mit einer getrennten Kontaktplatte,

Fig. 2 einen Ausschnitt aus einer Ausführungsform eines Fingerabdrucksensors, bei der ein Prisma eine aufgerauhte Seitenfläche aufweist,

Fig. 3 eine andere Ausführungsform eines Fingerabdrucksensors.

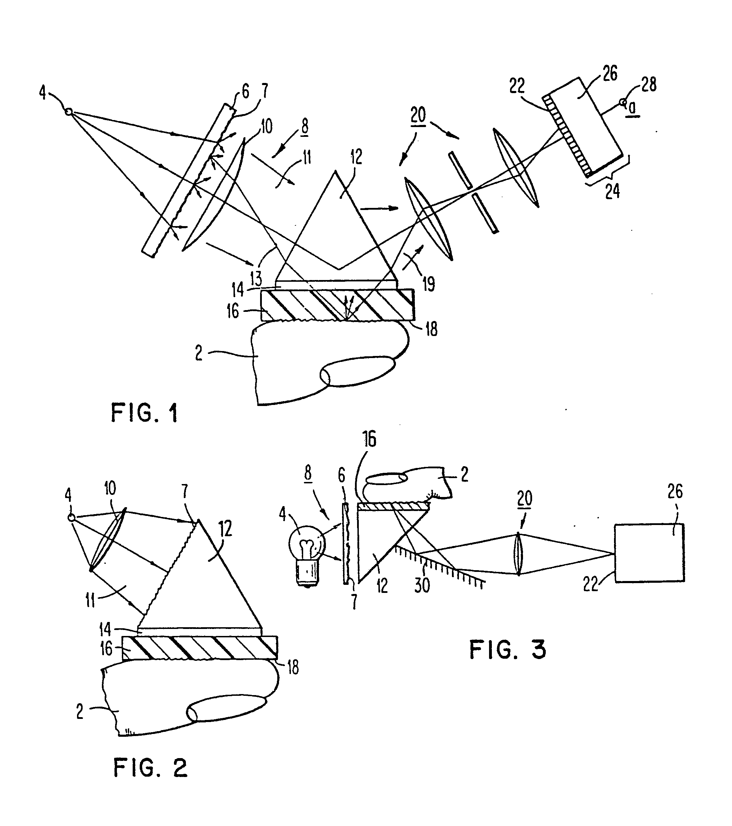

In der Figur 1 ist ein Fingerabdrucksensor zum Erzeugen eines dem topografischen Relief oder Muster eines zu untersuchenden Fingers entsprechenden Ausgangssignals a dargestellt.

Der Fingerabdrucksensor enthält eine Lichtquelle 4, die einen zum Abtasten des Fingerabdrucks dienenden Lichtstrahl aussendet. Die Lichtquelle 4 kann eine Weisslichtquelle, beispielsweise eine Glühlampe oder eine andere glühende Lichtquelle sein. Die Lichtquelle 4 kann auch aus einer lichtemittierenden Diode (LED) oder einem Halbleiterlaser bestehen, der Licht einer bestimmten Wellenlänge aussendet. Es ist jedoch als einer der Vorteile der Erfindung anzusehen, dass eine Lichtquelle verwendet werden kann, die ein breites Spektrum aussendet.

Das Licht aus der Lichtquelle 4 wird einem Lichtdiffusor 6 zugeführt. Der Diffusor 6 besteht aus einer Glasplatte, die eine aufgerauhte Oberfläche 7 aufweist. Das vom Diffusor 6 ausgesandte diffuse Licht wird von einem ersten optischen System 8 gesammelt, welches eine Kollimatorlinse 10 aufweist. Die Kollimatorlinse 10 führt einen Lichtstrahl 11 einem optischen Prisma 12 zu. Der Lichtstrahl 11 durchstrahlt die linke Seite des Prismas 12. Das Prisma 12 besteht vorzugsweise aus Glas. In der Figur 1 ist nur ein Teilstrahl 13 dargestellt, der in das Prisma 12 eindringt.

Ein beträchtlicher Anteil des einfallenden Lichtstrahls 11 trifft auf die Unterseite oder die plane Basisfläche des Prismas 12. Dort durchstrahlt er ein optisches Anpassungsmedium 14. Das Anpassungsmedium 14 besteht aus einer Substanz, die den Brechungsindex des Prismas 12 dem Brechungsindex eines benachbarten Kontaktkörpers anpasst. Dieser Kontaktkörper besteht aus einer Sensorplatte 16, die aus einem transparenten und elastischen Material gebildet ist. Dieses elastische Material kann ein Elastomer, beispielsweise ein Polymer sein. Vorzugsweise besteht das Material der Sensorplatte 16 aus Silikonkautschuk oder -gummi, der sich unter Druck verformt.

Die Sensorplatte 16 weist eine untere und obere planare Seite auf. Beide Seiten sind parallel zueinander. Die untere Seite bildet eine Kontaktfläche 18 zum Prüfen eines durch den Finger 2 auf sie ausgeübten Kontaktdruckes. Wenn der Finger 2 die Sensorplatte 16 nicht berührt, ist die Kontaktfläche 18 glatt. Wenn der Finger 2 auf die Kontaktfläche 18 gedrückt wird, ändert sich deren Struktur. Die Kontaktfläche 18 enthält dann ein Muster, welches dem topografischen Relief der Erhöhungen und Vertiefungen des Fingerabdrucks entspricht.

Das aus dem Prisma 12 kommende Licht durchstrahlt das Anpassungsmedium 14 und die Oberseite der Platte 16. Schliesslich trifft es auf die Kontaktfläche 18. Wenn die Kontaktfläche 18 von der Haut des zu überprüfenden Fingers 2 berührt wird, findet teilweise eine Spiegelreflexion statt.

Das von der Kontaktfläche 18 zurückkommende Licht ist mit der topologischen Struktur des Fingers 2 moduliert. Der Lichtstrahl 19 durchstrahlt das Anpassungsmaterial 14 und verlässt dann die rechte Seite des Prismas 12. Daraufhin durchstrahlt der Lichtstrahl 19 ein zweites optisches System 20.

Das zweite optische System 20 weist eine Abbildungslinse oder ein Linsensystem auf, sowie einige Elemente für eine optische Vorbehandlung, beispielsweise ein Farbfilter, welches nicht dargestellt ist. Der Lichtstrahl 19 durchstrahlt das zweite optische System 20 und trifft schliesslich auf ein lichtempfindliches Feld 22 eines Fotodetektors 24. Der Fotodetektor 24 misst die Verteilung des auf sein lichtempfindliches Feld 22 auftreffenden Lichts. Dieses Feld 22 kann aus einem Feld aus fotoempfindlichen Elementen gebildet sein. Die Ausgangssignale dieser Elemente werden einer digitalen Bildverarbeitungseinrichtung 26 zugeführt. Der Fotodetektor 24 ist im Hinblick auf das zweite optische System 20 so angeordnet, dass das Bild des Fingerabdrucks auf das lichtempfindliche Feld 22 fokussiert bzw. abgebildet wird. Das elektrische Ausgangssignal a ist dem Ausgang 28 des Fotodetektors 24 entnehmbar.

Wie oben erwähnt, kann ein Silikonpolymer oder -gummi als das transparente Wandlermaterial für die Sensorplatte 16 verwendet werden. Dieses Material ist in der Weise elastisch, dass ein Fingerabdruck in seine Oberfläche eindrückbar ist. Nach der Entfernung des Fingers erhält die Oberfläche wieder eine glatte und ebene Form. Für das Anpassungsmaterial 14 ist Silikonöl oder ein anderes optisches Öl geeignet. Diese Flüssigkeit lässt einen guten mechanischen Kontakt und eine gute optische Verbindung zwischen der Sensorplatte und dem Glasprisma 12 entstehen. Durch die Verwendung von Silikonöl oder einem anderen Anpassungsmedium 14 können vielfach Reflexionen vermieden werden.

Anstelle eines Silikonöls kann auch ein optischer Kitt mit einem geeignet gewählten Brechungsindex verwendet werden. Auch ein optischer Kleber mit den gleichen Eigenschaften kann zum Anbringen der Platte 16 an die Unterseite des Prismas 12 verwendet werden.

Bei einer nicht dargestellten anderen Ausführungsform kann das Sensormedium 16, beispielsweise ein Elastomer wie es Silikongummi darstellt, direkt die Unterseite des Prismas 12 bedecken. Bei dieser Ausführungsform ist ein Anpassungsmedium 14 nicht erforderlich.

Gemäss Figur 1 weist der Fotodetektor 14 ein lichtempfindliches Feld 22 und einen Signalverarbeitungsschaltkreis 26 auf. Der Fotodetektor 24 kann eine Standardvideokamera sein. Eine derartige Videokamera weist den Vorteil auf, dass sie an einen Mikrocomputer anschliessbar ist. Es ist jedoch auch möglich, eine im Handel erhältliche CCD-Matrix aus fotoempfindlichen Elementen zu verwenden. Eine derartige Matrix kann aus Gründen der Einfachheit und Zuverlässigkeit gewählt werden. Sie würde auch die Kosten und die Grösse des Fingerabdrucksensors reduzieren.

Es sei darauf hingewiesen, dass unter geeigneten Beleuchtungsvoraussetzungen und einer geeigneten Anordnung des Fotodetektors eine mit einem hohen Kontrast behaftete Ansicht des zu untersuchenden Fingerabdrucks erreicht werden kann.

In der Figur 2 ist eine andere Ausführungsform eines vorgeschlagenen Fingerabdrucksensors dargestellt. Bei dieser Ausführungsform wird ebenfalls ein Glasprisma 12 benutzt, jedoch fehlt ein Lichtdiffusor 6 gemäss Figur 1. An dessen Stelle ist die linke Seite des Prismas 12 aufgerauht. Dementsprechend ist auch bei dieser Ausführungsform ein als Lichtdiffusor wirkendes Feld 7 in dem Strahlengang zwischen der Lichtquelle 4 und der Kontaktfläche 18 angeordnet.

In der Figur 3 ist eine weitere Ausführungsform eines vorgeschlagenen Fingerabdrucksensors dargestellt. Bei dieser weiteren Ausführungsform wird ebenfalls ein Glasprisma 12 benutzt. Die beiden Seitenflächen des Prismas 12 sind im rechten Winkel zueinander angeordnet. Die linke Seite des Prismas 12 ist dem ersten optischen System 8 zugewandt, welches einen Lichtdiffusor 6 aufweist, wo hingegen die rechte Seite des Prismas 12 an der Kontakt- oder Sensorplatte 16 angebracht ist. Das von den Erhöhungen und Vertiefungen der Oberfläche der Platte 16 reflektierte Licht wird einem Spiegel 30 zugeführt, der Teil des zweiten optischen Systems 20 ist. Das zweite optische System 20 fokussiert oder bildet den Fingerabdruck auf das lichtempfindliche Feld 22 des Fotodetektors 26 ab.

## Patentansprüche

1. Fingerabdrucksensor zum Erzeugen eines dem Papillarmuster in der Haut eines Fingers (2) entsprechenden elektrischen Signals, mit einem Prisma (12) aus transparentem Material, auf einer dessen Seiten eine elastische Kontaktfläche (18) zum Aufdrücken des Fingers vorgesehen ist, in der das jeweils aufgedrückte Papillarmuster durch elastische Verformung ein entsprechendes topografisches Relief erzeugt, das mit einem der Kontaktfläche (18) durch das transparente Prisma (12) schräg zugeführten Lichtstrahl (13) aus einer Lichtquelle (4) beleuchtbar ist, wobei das beleuchtete Relief einen Lichtstrahl (9) schräg zur Kontaktfläche (18) reflektiert, der diesem Relief entsprechend moduliert ist und einem lichtempfindlichen Feld (22) einer optoelektrischen Fotodetektoreinrichtung (26) zur Erfassung der Verteilung der auf das Feld (22) auftreffenden Lichtintensität zugeführt ist, die ein dem topografischen Relief entsprechendes elektrisches Signal (a) erzeugt, das zugleich dem aufgedrückten Papillarmuster entspricht, dadurch gekennzeichnet, dass auf der einen Seite des inelastischen Prismas (12) eine Sensorplatte (16) aus einem transparenten elastischen Material aufgebracht ist, deren freiliegende Oberseite die Kontaktfläche (18) bildet, dass in dem Lichtstrahl (13) aus der Lichtquelle (4) vor der Kontaktfläche (18) ein Lichtdiffusor (6, 7) angeordnet ist und dass in dem von dem beleuchteten Relief reflektierten Lichtstrahl (19) vor dem lichtempfindlichen Feld eine Abbildungsoptik (20) angeordnet ist, welche das beleuchtete Relief (18) auf das lichtempfindliche Feld (22) abbildet.

2. Sensor nach Anspruch 1, dadurch gekennzeichnet, dass das Prisma (12) aus Glas ist.

3. Sensor nach Anspruch 1 oder 2, dadurch gekennzeichnet, dass zwischen der Sensorplatte (16) und dem Prisma (12) ein optisches Anpassungsmedium (14) angeordnet ist, das sowohl mit der Sensorplatte (16) als auch dem Prisma (12) in Kontakt steht.

4. Sensor nach einem der vorhergehenden Ansprüche, dadurch gekennzeichnet, dass der Lichtdiffusor (6, 7) aus einer Glasplatte (6) besteht, die eine aufgerauhte Oberfläche (7) aufweist.

5. Sensor nach einem der Ansprüche 1 bis 3, dadurch gekennzeichnet, dass der Lichtdiffusor aus einer rauhen Seitenfläche (7) des Prismas (12) besteht, durch die der das topografische Relief auf der Kontaktfläche (18) beleuchtende Lichtstrahl in das Prisma eintritt.

## Claims

1. A fingerprint sensor for producing an electrical signal corresponding to the papillary pattern in the skin of a finger (2), comprising a prism (12) of transparent material on one of whose sides is arranged an elastic contact surface (18) for impression by the finger, in which contact surface the respectively impressed papillary pattern produces a corresponding topographic relief by elastic deformation, which relief is illuminated by a light beam (13) transversely supplied to the contact surface (18) through the transparent prism (12) from a light source (4), the illuminated relief reflecting a light beam (9) transversely to the contact surface (18), which beam is modulated to correspond to said relief, which beam is supplied to a light-sensitive field (22) of an optoelectric photo detector device (26) for detecting the distribution of the light intensity incident on the field (22) and which produces an electrical signal (a) which corresponds to the topographic relief and simultaneously corresponds to the impressed papillary pattern, characterised in that on one side of the non-elastic prism (12) there is applied a sensor plate (16) of transparent elastic material whose exposed top side forms the contact surface (18), that a light diffusor (6, 7) is arranged in the light beam (13) from the light source (4) preceding the contact surface (18), and that in the light beam (19) reflected from the illuminated relief, preceding the light-sensitive field, there is arranged a focussing optical system (20) which focusses the illuminated relief (18) onto the light-sensitive field (22).

2. A sensor as claimed in Claim 1, characterised in that the prism (12) consists of glass.

3. A sensor as claimed in Claim 1 or 2, characterised in that an optical matching medium (14) is connected both to the sensor plate (16) and to the prism (12), being arranged between the sensor plate (16) and the prism (12).

4. A sensor as claimed in one of the preceding Claims, characterised in that the light diffusor (6, 7) consists of a glass plate (6) which has a roughened surface (7).

5. A sensor as claimed in one of Claims 1 to 3, characterised in that the light diffusor consists of a rough lateral surface (7) of the prism (12), through which the light beam which illuminates the topographic relief on the contact surface (18) enters the prism.

**Revendications**

1. Détecteur d'empreintes digitales servant à produire un signal électrique correspondant au modèle papillaire de la peau d'un doigt (2), comportant un prisme (12) constitué en un matériau transparent et sur une face duquel se trouve prévue une surface de contact élastique (18) prévue pour l'application du doigt et dans laquelle le modèle papillaire respectivement appuyé produit, par déformation élastique, un relief topographique correspondant qui peut être éclairé par un faisceau de lumière (13) délivré par une source de lumière (4) et envoyé obliquement sur la surface de contact (18), à travers le prisme transparent (12), le relief éclairé réfléchissant, obliquement par rapport à la surface de contact (18), un faisceau de lumière qui est modulé conformément à ce relief et est envoyé à une zone photosensible (22) d'un dispositif photodétecteur opto-électrique (26) pour la détection de la distribution de l'intensité de la lumière qui tombe sur la zone (22) et produit un signal électrique (a) correspondant au relief topographique et correspondant simultanément au modèle papillaire appliqué, caractérisé par le fait que sur une face du prisme inélastique (12) se trouve disposée une plaque de détection (16) constituée en un matériau élastique transparent, dont la face supérieure nue forme la surface de contact (18), qu'un diffuseur de lumière (6, 7) est disposé en avant de la surface de contact (18) dans le faisceau de lumière (13) délivré par la source de lumière (4) et que dans le faisceau de lumière (19) réfléchi par le relief éclairé se trouve disposé, en avant de la zone photosensible, un dispositif optique (20) de formation d'images, qui forme l'image du relief éclairé (18) sur la zone photosensible (22).

2. Détecteur suivant la revendication 1, caractérisé par le fait que le prisme (12) est en verre.

3. Détecteur suivant la revendication 1 ou 2, caractérisé par le fait qu'entre la plaque de détection (16) et le prisme (12) se trouve disposé un milieu d'adaptation optique (14) qui est en contact aussi bien avec la plaque de détection (16) qu'avec le prisme (12).

4. Détecteur suivant l'une des revendications précédentes, caractérisé par le fait que le diffuseur de lumière (6, 7) est constitué par une plaque de verre (6) qui possède une surface rugueuse (7).

5. Détecteur suivant l'une des revendications 1 à 3, caractérisé par le fait que le diffuseur de lumière est constitué par une surface latérale rugueuse (7) du prisme (12), au niveau de laquelle le faisceau de lumière, qui éclaire le relief topographique situé sur la surface de contact (18), pénètre dans le prisme.

FIG. 1

FIG. 2

FIG. 3